(19) Europäisches Patentamt
European Patent Office
Office européen des brevets

(11) Numéro de publication: **0 265 793**
**B1**

# (12) FASCICULE DE BREVET EUROPEEN

(45) Date de publication du fascicule du brevet: **03.01.90**

(21) Numéro de dépôt: **87115150.2**

(22) Date de dépôt: **06.04.84**

(60) Numéro de publication de la demande initiale en application de l'article 76 CBE: **0124407**

(51) Int. Cl.⁴: **C07C 59/64,** C07C 59/56, C07C 59/52, C07C 51/347, C07D 317/60

(54) **Procédé de préparation d'acides alkanoiques.**

(30) Priorité: **29.04.83 FR 8307137**

(43) Date de publication de la demande: **04.05.88 Bulletin 88/18**

(45) Mention de la délivrance du brevet: **03.01.90 Bulletin 90/1**

(84) Etats contractants désignés: **BE CH DE FR GB IT LI NL**

(56) Documents cités:
**EP-A- 0 032 374**
**FR-A- 2 415 109**
**FR-A- 2 470 127**

**Le dossier contient des informations techniques présentées postérieurement au dépôt de la demande et ne figurant pas dans le présent fascicule.**

(73) Titulaire: **SOCIETE FRANCAISE HOECHST Société anonyme dite:, 3, avenue du Général de Gaulle, F-92800 Puteaux(FR)**

(72) Inventeur: **Christidis, Yani, 53, boulevard de la Villette, F-75019 Paris(FR)**
Inventeur: **Vallejos, Jean-Claude, 9, rue Labat, F-75018 Paris(FR)**

(74) Mandataire: **Rinuy, Santarelli, 14, avenue de la Grande Armée, F-75017 Paris(FR)**

EP 0 265 793 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

La présente invention concerne un procédé de fabrication industrielle d'acides alkanoïques, plus particulièrement elle se rapporte à un procédé de fabrication d'acides acétiques substitués, ci-après désignés acides arylacétiques, de formule générale I,

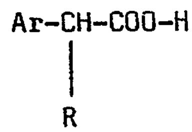

I

dans laquelle R représente un atome d'hydrogène ou un radical alkyle en $C_1$-$C_4$ et Ar représente un radical à caractère aromatique choisi parmi les radicaux suivants : méthoxy-2 naphtyle-1, méthylènedioxy-3, 4 phényle et phényles substitués de formule générale II,

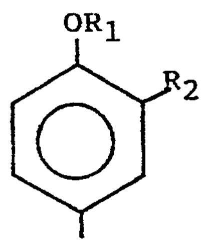

II

où $R_1$ représente un atome d'hydrogène ou un groupement alkyle en $C_1$-$C_4$ et $R_2$ représente un atome d'hydrogène, un atome d'halogène, un groupement alkyle, alcoxyle ou hydroxyle. Les acides arylacétiques de formule générale I sont largement décrits dans la littérature. Ce sont notamment des matières premières intéressantes pour la synthèse de substances pharmaceutiques actives. Il est donc d'un grand intérêt de pouvoir les préparer d'une manière aussi rationnelle que possible.

On connaît de très nombreuses méthodes pour préparer des acides acétiques substitués. Parmi celles-là, la réaction de Willgerodt-Kindler qui autorise la transformation d'une arylalkylcétone en acide arylalkylcarboxylique est l'une des plus connues. Toutefois, elle ne fournit généralement que des rendements modérés à médiocres et elle conduit à des produits secondaires soufrés responsables d'une pollution inacceptable aujourd'hui. Pour remédier à ces inconvénients, on s'est adressé à des méthodes plus élaborées nécessitant l'emploi de plusieurs stades réactionnels tels que notamment l'hydroénolyse, soit chimique, soit catalytique, d'acides arylglycoliques, d'acides o-acylé arylglycoliques ou d'acides aryl-2 halogéno-2 alkanoïques, issus dans certains cas de la condensation de l'acide glyoxylique sur le dérivé aromatique correspondant (demandes de brevet européen 0.032.374 et 0.028.375, brevet européen 0.003.825), l'hydrolyse d'un arylalkylacétonitrile obtenu par réaction de l'ion cyanure sur une benzylamine quaternisée convenablement subsituée (demande de brevet européen 0.062.440). Ces méthodes, dont certains sont très récentes, exigent comme matière première de départ des produits rarement disponibles sur le marché ce qui nécessite leur préparation préalable avec les inconvénients que cela comporte.

Or, la Demanderesse a découvert avec étonnement qu'il était possible d'obtenir des acides arylacétiques de formule générale I, en un seul stade, par réaction à chaud, en milieu acide, en présence de phosphore rouge et de quantités catalytiques d'iode ou d'acide iodhydrique, d'un acide carboxylique alpha carbonylé de formule générale III, R-CO-COOH, dans laquelle R a la signification donnée précédemment avec un dérivé insaturé à caractère aromatique choisi parmi les suivants: méthoxy-2 naphtalène, méthylènedioxy-1,2 benzène ou benzodioxole-1,3 et les hydrocarbures aromatiques substitués de formule générale IV, dans laquelle $R_1$ et $R_2$ ont la signification donnée précédemment :

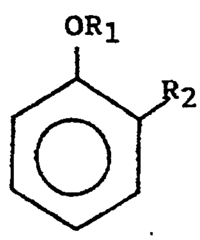

IV

Par la suite, cet ensemble de produits insaturés à caractère aromatique sera désigné par A.

Malgré que corrélativement à la formation d'une mole d'acide arylacétique de formule générale I, il se

forme une mole d'eau, il est parfois avantageux de mettre en oeuvre le procédé de la présente invention en présence de faibles quantités d'eau supplémentaires.

Plus particulièrement, le procédé selon la présente invention consiste à faire réagir, à une température comprise entre 30 et 100°C, en milieu acide, une mole d'un acide carboxylique alpha carbonylé, de formule générale III, avec de une à dix moles d'un dérivé appartenant à l'ensemble A, en présence de 0,5 à 5 atome-grammes de phos phore rouge, de 0,5 à 2 moles d'eau et de 0,01 à 0,1 mole d'iode ou d'acide iodhydrique, éventuellement au sein d'un solvant organique compatible tel que l'acide acétique, puis à isoler l'acide cherché de formule générale I par des moyens connus en soi.

Avantageusement, le procédé selon la présente invention consiste à faire réagir, à une température comprise entre 50 et 80°C, en milieu acide, une mole d'acide glyoxylique ou d'acide pyruvique, avec de une à dix moles d'un dérivé appartenant à l'ensemble A en présence de 0,5 à 3 atome-grammes de phosphore rouge, de 0,5 à 2 moles d'eau et de 0,025 à 0,05 mole d'iode ou d'acide iodhydrique éventuellement au sein d'acide acétique, puis à isoler l'acide cherché de formule I, par des moyens connus en soi.

L'acide iodhydrique est l'acide iodhydrique commercial en solution aqueuse à 57 % en poids.

Selon une variante, on peut mettre en oeuvre le procédé de la présente invention en présence de 0,001 à 1 mole, avantageusement de 0,005 à 0,3 mole d'un acide organique sulfonique tel que l'acide méthanesulfonique par mole d'acide carboxylique alpha carbonylé de formule générale III, engagé. La durée réactionnelle du procédé de la présente invention est fonction des substrats mis en oeuvre ; habituellement cette durée est comprise entre 4 et 20 heures.

Préférentiellement, l'acide carboxylique alpha carbonylé de formule générale III est l'acide glyoxylique ou l'acide pyruvique. L'acide glyoxylique utilisé est généralement de l'acide glyoxylique en solution aqueuse à 80 % en poids.

En fin de réaction, l'acide arylacétique cherché de formule générale I est isolé du milieu réactionnel par des moyens connus en soi. Généralement, on élimine du milieu réactionnel ramené à la température ambiante le phosphore non transformé par filtration, puis on traite le filtrat avec de l'acétate de sodium pour neutraliser l'acide iodhydrique et/ou l'acide organique sulfonique éventuellement présents.

Le filtrat est ensuite concentré sous vide à température contrôlée pour n'éliminer que le solvant réactionnel et/ou le produit de départ qui n'a pas réagi. Le concentré ainsi obtenu est dissous dans un solvant organique compatible, non miscible à l'eau et la solution obtenue est lavée d'abord avec de l'eau contenant en solution du métabisulfite de sodium puis à l'eau, enfin elle est concentrée à sec sous vide. On isole ainsi l'acide arylacétique cherché de formule générale I qui pourra être si nécessaire purifié par recristallisation.

Avec certains substrats, il est parfois avantageux d'isoler l'acide arylacétique cherché sous forme de sel sodique, puis de déplacer ce sel par de l'acide chlorhydrique concentré.

Le procédé de la présente invention permet d'obtenir, entre autres, les acides suivants :
- acide paraméthoxyphénylacétique ;
- acide diméthoxy-3,4 phénylacétique ;
- acide chloro-3 hydroxy-4 phénylacétique ;
- acide méthylènedioxy-3,4 phénylacétique ;
- acide (méthoxy-2 naphtalène-1) acétique ;
- acide méthyl-2 parahydroxyphényl-2 acétique.

Improcédé analogue pour la synthése de l'acide thiényl-2 acétique est décrit dans EP-A 124 407.

Tous ces acides sont des matières premières précieuses pour la préparation de substances à activités thérapeutiques.

Les exemples suivants sont donnés à titre explicatif et nullement limitatif de l'invention.

<u>Exemple 1</u> :

On chauffe pendant 390 minutes à 70°C sous agitation une suspension de :
- 277,5 g (3 moles) d'acide glyoxylique à 80 % en poids dans l'eau ;
- 972 g (9 moles) d'anisole ;
- 21 g (0.0827 mole) d'iode bisublimé pur ;
- 279 g (9 at. g.) de phosphore rouge ;
- 600 cm3 d'acide acétique pur cristallisable.

Puis le milieu réactionnel refroidi à la température ambiante est filtré pour éliminer le phosphore qui n'a pas réagi. Le filtrat traité par 14 g (0,17 mole) d'acétate de sodium pur sec est ensuit concentré sous un vide de 20 mm Hg. Le résidu est repris par 400 cm3 d'eau et 600 cm3 d'éther éthylique. On décante et la phase éthérée est lavée d'abord avec une solution aqueuse à 5 % en poids de métabisulfite de sodium puis à l'eau. La phase organique est ensuite concentrée à sec sous vide et le résidu huileux (455 g) est distillé sous vide. On recueille ainsi 368,8 g (2,22 moles) d'acide paraméthoxyphénylacétique distillant à 180 ± 3°C sous un vide de 3 mm Hg et présentant un point de fusion de 85 - 1°C. le rendement s'établit à 74 % de la théorie calculée par rapport à l'acide glyoxylique mis en oeuvre.

Exemple 2 :

On chauffe pendant 16 heures à 70°C sous agitation une suspension de :
- 46,3 g (0,5 mole) d'acide glyoxylique à 80 % en poids dans l'eau ;
- 183 g (1,5 mole) de méthylènedioxy-1,2 benzène ;
- 46,5 g (1,5 at.g.) de phosphore rouge ;
- 3,3 cm3 (0,025 mole) d'acide iodhydrique à 57 %, d = 1,7 ;
- 10 g (0,102 mole) d'acide méthanesulfonique à 98 % ;
- 250 cm3 d'acide acétique.

Puis le milieu réactionnel refroidi à la température ambiante est filtré pour éliminer le phosphore qui n'a pas réagi. Le filtrat traité par 10,5 g (0,128 mole) d'acétate de sodium pur sec est ensuite concentré à sec sous vide. Le résidu huileux est repris avec un mélange d'eau et d'éther sulfurique. On décante et on lave la phase éthérée avec une solution aqueuse de métabisulfite de sodium puis à l'eau.

Par concentration à sec sous vide de la phase organique, on recueille 84,5 g de produit partiellement cristallisé qui cristallise par chaud et froid dans 6 volumes d'un mélange acide acétique-eau 1/4 v/v. On isole ainsi 53,6 g (0.2976 mole) d'acide méthylènedioxy-3,4 phénylacétique cristallisé présentant un point de fusion de 127 ± 1°C. Le rendement s'établit à 59,5 % de la théorie calculée par rapport à l'acide glyoxylique mis en oeuvre.

Exemple 3 :

On chauffe pendant 4 heures à 80°C sous agitation une suspension de :
- 46,3 g (0,5 mole) d'acide glyoxylique à 80 % en poids dans l'eau ;
- 80,35 g (0,625 mole) d'orthochlorophénol ;
- 1,65 cm3 (0,0125 mole) d'acide iodhydrique à 57 %, d = 1,7 ;
- 5 g (0,05 mole) d'acide méthanesulfonique à 98 % ;
- 19 g (0,6 at.g.) de phosphore rouge.

Puis le milieu réactionnel est versé dans 100 g d'eau glacée ; la suspension obtenue est filtrée et le filtrat est traité par 30 cm3 de soude à 30 % (0,3 mole) et ensuite il est soumis à un entraînement à la vapeur d'eau pour éliminer l'orthochlorophénol qui n'a pas réagi. On récupère ainsi 32 g d'orthochlorophénol soit 0,25 mole. Le résidue d'entraînement à la vapeur d'eau est amené à pH = 7 avec de la soude à 30 %. Le sel de sodium de l'acide chloro-3 hydroxy-4 phénylacétique cristallise. On l'essore puis on le sèche sous vide à 110°C à poids constant. On isole ainsi 66 g de chloro-3 hydroxy-4 phénylacétate de sodium cristallisé. Ce sol est mis en suspension dans un volume d'eau à 50°C ; on introduit ensuite de l'acide chlorohydrique concentré jusqu'à obtention d'un pH = 1. Il se forme une huile qui cristallise lentement. On essore le produit cristallisé, puis on le sèche sous vide à 70°C à poids constant. On recueille 53,5 g (0.286 mole) d'acide chloro-3 hydroxy-4 phénylacétique, cristallisé présentant un point de fusion de 108 ± 1°C. le rendement s'établit à 57,3 % de la théorie calculée par rapport à l'acide glyoxylique mis en oeuvre.

Exemple 4 :

On chauffe sous agitation pendant 16 heures à 70°C une suspension de :
- 46,3 g (0,5 mole) d'acide glyoxylique à 80 % dans l'eau ;
- 69 g (0,5 mole) de vératrole ;
- 3,3 cm3 (0,025 mole) d'acide iodhydrique à 57 %, d = 1,7 ;
- 10 g (0,1 mole) d'acide méthanesulfonique à 98 % ;
- 46,5 g (1,5 at.g.) de phosphore rouge ;
- 250 cm3 d'acide acétique.

Puis on traite le milieu réactionnel comme décrit à l'exemple 2. On recueille ainsi 68,7 g d'acide diméthoxy-3,4 phénylacétique brut que l'on purifie par dissolution dans 525 cm3 d'eau contenant 17,5 g d'hydroxyde de sodium, lavage de la solution aqueuse résultante avec de l'éther sulfurique puis précipitation de l'acide cherché par addition d'acide chlorhydrique concentré jusqu'à pH : 1. On isole ainsi 48,1 g (0.245 mole) d'acide diméthoxy-3,4 phénylacétique cristallisé présentant un point de fusion de 97 ± 1°C. Le rendement s'établit à 49 % de la théorie calculée par rapport à l'acide glyoxylique mis en oeuvre.

Exemple 5 :

On chauffe 8 heures à 75°C, sous agitation, une suspension de :
- 46,3 g (0,5 mole) d'acide glyoxylique à 80 % en poids dans l'eau ;
- 237,5 g (1,5 mole) de méthoxy-2-naphtalène ;
- 5 g (0,02 mole) d'iode bisublimé pur ;
- 46,5 g (1,5 at.g.) de phosphore rouge ;
- 175 cm3 d'acide acétique pur cristallisable.

Puis le milieu réactionnel est refroidi à la température ambiante. Il se prend en masse. On le reprend par 650 cm3 d'acétate d'éthyle au reflux, on filtre à chaud et le filtrat est concentré à sec sous vide. Le

résidu est repris par de l'éther sulfurique et par une solution aqueuse saturée en bicarbonate de sodium. On décante et la phase aqueuse est acidifiée à pH : 1 avec de l'acide chlorhydrique concentré. Le produit cherché cristallise. On l'essore puis on le sèche sous vide à 100°C à poids constant. On isole ainsi 20 g (0,0925 mole) d'acide méthoxy-2 naphtalèneacétique cristallisé présentant un point de fusion de 216 ± 1°C. Le rendement s'établit à 18,5 % de la théorie calculée par rapport à l'acide glyoxylique mis en oeuvre.

Exemple 6 :

On chauffe durant 7 heures à 75°C sous agitation une suspension de :
- 44 g (0,5 mole) d'acide pyruvique ;
- 70,5 g (0,75 mole) de phénol ;
- 3,3 cm3 (0,025 mole) d'acide iodhydrique à 57 %, d = 1,7 ;
- 4,9 g (0,05 mole) d'acide méthanesulfonique à 98 %.
- 23 g (0,74 at. g.) de phosphore rouge ;
- 50 cm3 d'acide acétique pur cristallisable ;
- 10 g d'eau.

Puis le milieu réactionnel refroidi à la température ambiante est filtré. Le filtrat traité avec 6,5 g (0,08 mole) d'acétate de sodium pur sec est ensuite concentré à sec. Le résidu est repris avec 400 g d'eau et suffisamment de soude à 20 % en poids pour obtenir une solution présentant un pH de 6. Cette solution est lavée à l'éther éthylique puis acidifiée à pH : 1,5 avec de l'acide chlorohydrique concentré.

L'acide cherché est ensuite extrait à l'éther éthylique puis isolé à l'état brut par élimination sous vide du solvant d'extraction.

On isole ainsi 40 g d'acide brut cristallisé que l'on purifie par distillation sous vide ($E_{0,1}$ = 150 - 4°C). Par refroidissement, le distillat cristallise. On obtient ainsi l'acide parahydroxyphényl-2 méthyl-2 acétique présentant un point de fusion de 130 ± 2°C.

Il va de soi que la présente invention n'a été décrite qu'à titre purement explicatif et nullement limitatif et que modification utile pourra y être apportée sans sortir de son cadre.

## Revendications

1. Procédé de préparation en un seul stade d'acides acétiques substitués de formule générale I :
Ar - CHR - COOHI
dans laquelle R représente un atome d'hydrogène ou un radical alkyle en C!-C$ et Ar représente un radical à caractère aromatique choisi parmi les radicaux suivants : méthoxy-2 naphtyle-1, méthylènedioxy-3,4 phényle, et les phényles substitués de formule générale II :

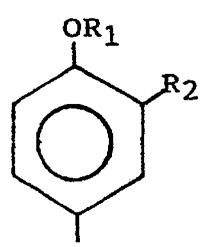

II

où R! représente un atom d'hydrogène ou un groupement alkyle en C!-C$ et R@ représente un atome d'hydrogène, un atome d'halogène, un groupement alkyle, alkoxyle ou hydroxyle, par réaction à chaud, en milieu acide en présence de phosphore rouge et d'iode ou d'acide iodhydrique, d'un acide carboxylique alpha carbonylé de formule générale III :
R- CO - COOHIII
dans laquell e R a la signification ci-dessus, avec un dérivé insaturé à caractère aromatique chosi parmi les suivants : méthoxy-2 naphtalène, méthylènedioxy-1,2 benzène et les hydrocarbures aromatiques substitués de formule générale IV :

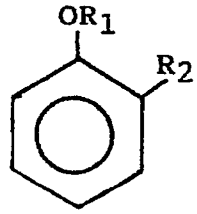

IV

dans laquelle $R_1$ et $R_2$ ont la signification ci-dessus, éventuellement au sein d'un solvant organique compatible, procédé caractérisé par le fait que l'iode ou l'acide iodhydrique sont utilisés en quantités catalytiques en présence ou non d'un acide organique sulfonique.

2. Procédé selon la revendication 1, caractérisé par le fait que l'on fait réagir à une température comprise entre 30 et 100°C, en milieu acide, une mole d'acide carboxylique alpha carbonylé de formule générale III telle que définie à la revendication 1, avec de 1 à 10 moles d'un dérivé insaturé à caractère aromatique tel que défini à la revendication 1, en présence de 0,5 à 5 atome-grammes de phosphore rouge, de 0,5 à 2 moles d'eau et de 0,01 à 0,1 mole d'iode ou d'acide iodhydrique, éventuellement au sein d'acide acétique.

3. Procédé selon l'une quelconque des revendications 1 ou 2, caractérisé par le fait que l'on fait réagir à une température comprise entre 50 et 80°C, en milieu acide, une mole d'acide glyoxylique ou d'acide pyruvique avec de 1 à 10 moles d'un dérivé insaturé à caractère aromatique tel que défini à la revendication 1, en présence de 0,5 à 3 atome-grammes de phosphore rouge, de 0,5 à 2 moles d'eau et de 0,025 à 0,05 mole d'iode ou d'acide iodhydrique, éventuellement au sein d'acide acétique.

4. Procédé selon l'une quelconque des revendications 1 à 3, caractérisé par le fait qu'il est réalisé en présence de 0,001 à 1 mole d'un acide organique sulfonique par mole d'acide carboxylique alpha carbonylé mis en oeuvre.

5. Procédé selon la revendication 4, caractérisé par le fait que l'acide organique sulfonique utilisé est l'acide méthanesulfonique.

6. Application du procédé selon l'une quelconque des revendications 1 à 5 à la fabrication de l'acide paraméthoxyphénylacétique, caractérisée par le fait que le dérivé insaturé à caractère aromatique mis en oeuvre est l'anisole et que l'acide carboxylique alpha carbonylé utilisé est l'acide glyoxylique.

7. Application du procédé selon l'une quelconque des revendications 1 à 5 à la fabrication de l'acide chloro-3 hydroxy-4 phénylacétique, caractérisé par le fait que le dérivé insaturé à caractère aromatique mis en oeuvre est l'orthochlorophénol et que l'acide carboxylique alpha carbonylé utilisé est l'acide glyoxylique.

8. Application du procédé selon l'une quelconque des revendications 1 à 5 à la fabrication de l'acide méthyl-2 parahydroxyphénylacétique, caractérisée par le fait que le dérivé insaturé à caractère aromatique mis en oeuvre est le phénol et l'acide carboxylique alpha carbonylé utilisé est l'acide pyruvique.

**Claims**

1. A process for the single-stage preparation of substituted acetic acids having the general formula I:

Ar–CHR–COOH

where R denotes a hydrogen atom or a $C_1$-$C_4$ alkyl radical and Ar denotes a radical of aromatic nature selected from the following radicals: 2-methoxy 1–naphthyl, 3,4-methylenedioxy phenyl, and substituted phenyls having the general Formula II:

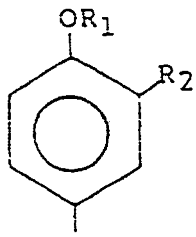

II

where $R_1$ denotes a hydrogen atom or a $C_1$-$C_4$ alkyl, alkoxyl or hydroxyl group, by reacting hot, in an acid medium in the presence of red phosphorus and iodine or hydriodic acid, a carbonylated alpha carboxylic acid having the general Formula III:

R–CO–COOH

where R has the above meaning, with an unsaturated derivative of aromatic nature selected from the following: 2-methoxy naphthalene, 1, 2-methylenedioxy benzene and substituted aromatic hydrocarbons having the general Formula IV:

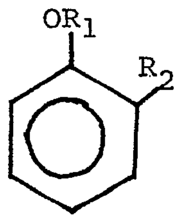

IV

where $R_1$ and $R_2$ have the above meanings, if necessary in a compatible organic solvent, the process being characterized in that the iodine or hydriodic acid are used in catalytic quantities in the presence or not of a sulphonic organic acid.

2. A process according to claim 1, characterized in that one mole of carbonylated alpha carboxylic acid having the general Formula III, as defined in claim 1, is reacted at a temperature between 30 and 100°C in an acid medium with 1 to 10 moles of an unsaturated derivative of aromatic nature, as defined in claim 1, in the presence of 0.5 to 5 gram-atoms of red phosphorus, 0.5 to 2 moles of water and 0.01 to 0.1 mole of iodine or hydriodic acid, possibly in acetic acid.

3. A process according to either of claims 1 or 2, characterized in that one mole of glyoxylic acid or pyruvic acid is reacted at a temperature between 50 and 80°C in an acid medium with 1 to 10 moles of an unsaturated derivative of aromatic nature, as defined in claim 1, in the presence of 0,5 to 3 gram-atoms of red phosphorus, 0.5 to 2 moles of water and 0.025 to 0.05 mole of iodine or hydriodic acid, possibly in acetic acid.

4. A process according to any of claims 1 to 3, characterized in that it is performed in the presence of 0.001 to 1 mole of a sulphonic organic acid per mole of carbonylated alpha carboxylic acid used.

5. A process according to claim 4, characterized in that the sulphonic organic acid used is methane sulphonic acid.

6. Application of the process according to any of claims 1 to 5 to the preparation of paramethoxy phenyl acetic acid, characterized in that the unsaturated derivative of aromatic nature used is anisole, and the carbonylated alpha carboxylic acid used is glyoxylic acid.

7. Application of the process according to any of claims 1 to 5 to the preparation of 3-chloro-4-hydroxy phenyl acetic acid, characterized in that the unsaturated derivative of aromatic nature used is orthochloro phenol, and the carbonylated alpha carboxylic acid used is glyoxylic acid.

8. Application of the process according to any of claims 1 to 5 to the preparation of 2-methyl parahydroxy phenyl acetic acid, characterized in that the unsaturated derivative of aromatic nature used is phenol, and the carbonylated alpha carboxylic acid used is pyruvic acid.

**Patentansprüche**

1. Verfahren zur einstufigen Herstellung von substituierten Essigsäuren der allgemeinen Formel I

Ar–CHR–COOH

in der R ein Wasserstoffatom oder einen $C_{1-4}$-Alkylrest und Ar einen Rest aromatischen Charakters bedeutet, der aus den Resten Methoxy-2-naphtyl-1, Methylendioxy-3,4-phenyl und den substituierten Phenylen der allgemeinen Formel II

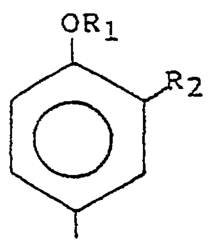

II

ausgewählt wird, in der $R_1$ ein Wasserstoffatom oder eine $C_{1-4}$-Alkylgruppe und $R_2$ ein Wasserstoffatom, ein Halogenatom, eine Alkyl-, Alkoxyl- oder eine Hydroxylgruppe darstellt, durch in Wärme sowie in sauerem Milieu in Gegenwart von rotem Phosphor und Jod oder Jodwasserstoffsäure vorgenommener Umsetzung einer alpha-carbonylierten Carbonsäure der allgemeinen Formel III

R–CO–COOH

in der R die vorstehend angegebene Bedeutung hat, mit einem ungesättigten Derivat aromatischen Charakters, das aus den Resten Methoxy-2-naphtalen, Methylendioxy-1,2-benzol und den substituierten aromatischen Kohlenwasserstoffen der allgemeinen Formel IV

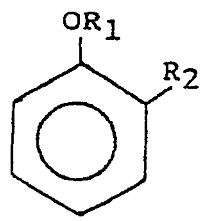

IV

ausgewählt wird, in der $R_1$ und $R_2$ die vorstehende Bedeutung haben, gegebenenfalls in einem verträglichen organischen Lösungsmittel, dadurch gekennzeichnet, daß das Jod oder die Jodwasserstoffsäure in katalytischen Mengen zusammen mit einer organischen Sulfonsäure oder ohne diese eingesetzt wird.

2. Verfahren nach Anspruch 1, dadurch gekennzeichnet, daß man bei einer Temperatur von 30 bis 100°C in sauerem Milieu ein Mol alpha-carbonylierte Carbonsäure der allgemeinen Formel III, wie in Anspruch 1 angegeben, mit 1 bis 10 Molen eines ungesättigten Derivats aromatischen Charakters, wie in Anspruch 1 angegeben, zusammen mit 0,5 bis 5 Grammatomen roten Phosphors, 0,5 bis 2 Molen Wasser und 0,01 bis 0,1 Mol Jod oder Jodwasserstoffsäure, gegebenenfalls in Essigsäure, umsetzt.

3. Verfahren nach einem der Ansprüche 1 oder 2, dadurch gekennzeichnet, daß man bei einer Temperatur von 50 bis 80°C in saurem Milieu ein Mol Glyoxalsäure oder Brenztraubensäure mit 1 bis 10 Molen eines ungesättigten Derivats aromatischen Charakters, wie in Anspruch 1 angegeben, zusammen mit 0,5 bis 3 Grammatomen roten Phosphors, 0,5 bis 2 Molen Wasser und 0,025 bis 0,05 Molen Jod oder Jodwasserstoffsäure, gegebenenfalls in Essigsäure, umsetzt.

4. Verfahren nach einem der Ansprüche 1 bis 3, dadurch gekennzeichnet, daß es in Gegenwart von 0,001 bis 1 Mol einer organischen Sulfonsäure pro Mol eingesetzter alpha-carbonylierter Carbonsäure durchgeführt wird.

5. Verfahren nach Anspruch 4, dadurch gekennzeichnet, daß die verwendete organische Sulfonsäure Methansulfonsäure ist.

6. Anwendung des Verfahrens nach einem der Ansprüche 1 bis 5 zur Herstellung von Paramethoxyphenylessigsäure, dadurch gekennzeichnet, daß das eingesetzte ungesättigte Derivat aromatischen Charakters Anisol und die verwendete alpha-carbonylierte Carbonsäure Glyoxalsäure ist.

7. Anwendung des Verfahrens nach einem der Ansprüche 1 bis 5 zur Herstellung von Chlor-3-hydroxy-4-phenylessigsäure, dadurch gekennzeichnet, daß das eingesetzte ungesättigte Derivat aromatischen Charakters Orthochlorphenol und die verwendete alpha-carbonylierte Carbonsäure Glyoxalsäure ist.

8. Anwendung des Verfahrens nach einem der Ansprüche 1 bis 5 zur Herstellung von Methyl-2-parahydroxyphenylessigsäure, dadurch gekennzeichnet, daß das eingesetzte ungesättigte Derivat aromatischen Charakters Phenol und die verwendete alpha-carbonylierte Carbonsäure Brenztraubensäure ist.